# EUROPEAN PATENT APPLICATION

(11) **EP 4 148 125 A1**
(43) Date of publication of application: **15.03.2023**
(21) Application number: 21842341.6
(22) Date of filing: 14.07.2021
(51) Int. Cl.: C12N 5/10, C12N 15/113, A61P 35/00, A61K 35/17, A61P 35/02

(54) **ENGINEERED IMMUNE CELL FOR ALLOTRANSPLANTATION**

(30) Priority: 15.07.2020 CN 202010679530; 22.12.2020 CN 202011534699
(71) Applicant: Nanjing Bioheng Biotech Co., Ltd, Nanjing, Jiangsu 210061 (CN)
(72) Inventor: ZHOU, Yali, Nanjing, Jiangsu 210061 China Jiangsu 210061 (CN); CHEN, Gong, Nanjing, Jiangsu 210061 China Jiangsu 210061 (CN); JIANG, Xiaoyan, Nanjing, Jiangsu 210061 China Jiangsu 210061 (CN); REN, Jiangtao, Nanjing, Jiangsu 210061 China Jiangsu 210061 (CN); HE, Xiaohong, Nanjing, Jiangsu 210061 China Jiangsu 210061 (CN); WANG, Yanbin, Nanjing, Jiangsu 210061 China Jiangsu 210061 (CN); HAN, Lu, Nanjing, Jiangsu 210061 China Jiangsu 210061 (CN)
(74) Representative: Comoglio, Elena
(86) International application number: PCT/CN2021/106259
(87) International publication number: WO 2022/012591

(57) **Abstract**

Provided is an engineered immune cell. The expressions of at least one MHC related gene and at least one NK activating receptor binding molecule are suppressed or silenced, so as to suppress the killing of the engineered immune cell by NK cells. Also provided are a pharmaceutical composition comprising the engineered immune cell and use of the engineered immune cell in preparation of drugs for treatment of cancer, infection, or autoimmune diseases.

## Description

### Technical Field

The present disclosure belongs to the field of immunotherapy. More specifically, the present disclosure relates to an engineered immune cell, in which expressions of at least one NK activating receptor binding molecule and at least one MHC related gene are suppressed or silenced, so as to suppress the killing of the engineered immune cell by NK cells.

### Background Art

In recent years, cancer immunotherapy technology has been rapidly developed, and particularly immunotherapy associated with chimeric antigen receptor T cell (CAR-T) has achieved excellent clinical effects in the treatment of hematologic tumors. CAR-T cell immunotherapy is to genetically modify T cells *in vitro* to enable the T cells to recognize tumor antigens and the T cells, after being amplified to a certain amount, are infused back into the patients' body to kill the cancer cells, thus achieving the purpose of treating tumors.

In 2017, two autologous CAR-T therapies were approved by the FDA and launched in the US, one for B-cell acute leukemia and the other for diffuse B-cell non-Hodgkin's lymphoma. Although these two CAR-T cells have excellent clinical treatment effect, they are expensive and have a long preparation cycle, making large-scale promotion quite difficult. Therefore, it is necessary to develop a universal CAR-T product that can be used for allotransplantation (allogeneic transplantation) so as to solve the above problem. Universal CAR-T can be prepared by using T cells isolated from peripheral blood of healthy donors, thereby realizing allogeneic transfusion, and greatly shortening the waiting time of a patient for treatment. Besides, the viability and function of T cells obtained from healthy donors are also superior to those of patient-derived T cells, which can increase the CAR infection rate and improve the therapeutic effect.

However, the development of universal CAR-T cells still faces two problems: (1) after the engineered CAR-T cells enter a patient's body and proliferate to a certain extent, they may attack normal cells or tissues of the patient, thus generating graft-versus-host disease (GvHD); and (2) the normal immune system in the patient's body may reject exogenous CAR-T cells, thus causing host-versus-graft disease (HvGD). Transplantation risk is currently reduced primarily by knocking out TCR, MHC class I molecules and/or class II molecules (e.g., HLA, B2M etc.). However, since MHC class molecules are key molecules for identifying "autologous cells" by NK cells, complete inactivation of MHC class I molecules such as B2M can cause the NK cells to recognize the transfused engineered immune cells as "allogeneic" and kill the same, finally affecting survival and persistence of the transfused cells, and further influencing the therapeutic effect.

Therefore, there is a need to develop a novel engineered immune cell, which can reduce or suppress the killing effect of NK cells in a recipient's body on the same, and further reduce or avoid the risk of immunological rejection caused by allotransplantation.

### Summary

In a first aspect, the present disclosure provides an engineered immune cell in which expressions of at least one MHC related gene and at least one NK activating receptor binding molecule are suppressed or silenced, so as to suppress the killing of the engineered immune cell by NK cells.

In an embodiment, the MHC related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA and a combination thereof, preferably from HLA-A, HLA-B, HLA-C, B2M, RFX5, RFXAP, RFXANK, CIITA and a combination thereof.

In an embodiment, the NK activating receptor binding molecule binds to an NK activating receptor selected from the group consisting of NKG2C, NKG2E, NKG2D, NKp30, NKp44, NKp46, NKp80, 2B4, DNAM-1, CD2 and LFA-1. Preferably, the NK activating receptor binding molecule binds to an NK activating receptor selected from the group consisting of NKG2D, Nkp30, 2B4, DNAM-1, CD2 and LFA-1.

In an embodiment, the NK activating receptor binding molecule is selected from the group consisting of MICA, MICB, ULBP1, ULBP2, ULBP3, ULBP4, ULBP5, ULBP6, Rae-1, H60, MULT1, B7-H6, BAG6, PfEMP1, HSPGS, AICL, CD112, CD155, CD48, CD58, CD59, ICAM1, ICAM2, ICAM3, STAT1, JAK1, IFNGR2, JAK2 and IFNGR1. Preferably, the NK activating receptor binding molecule is selected from the group consisting of CD112, CD155, CD48, MICA, MICB, CD58, B7-H6, ICAM1, ICAM2 and ICAM3.

In an embodiment, the engineered immune cell further comprises suppressed or silenced expression of at least one TCR/CD3 gene selected from the group consisting of TRAC, TRBC, CD3 γ, CD3 δ, CD3 ε, CD3 ζ and a combination thereof.

In a preferred embodiment, expressions of at least one TCR/CD3 gene, at least one MHC related gene, and at least one NK activating receptor binding molecule of the engineered immune cell are suppressed or silenced, wherein the TCR/CD3 gene is selected from the group consisting of TRAC, TRBC and a combination thereof, the MHC related gene is selected from the group consisting of B2M, RFX5, RFXAP, RFXANK, CIITA and a combination thereof, and the NK activating receptor binding molecule is selected from the group consisting of CD112, CD155, CD48, MICA/B, ULBP1, ULBP2, ULBP3, ULBP4, ULBP5, ULBP6, CD58, B7-H6, ICAM1, ICAM2 and ICAM3. In an embodiment, the expression of TRAC or TRBC, B2M, and at least one NK activating receptor binding molecule of the engineered immune cell is suppressed or silenced. In an embodiment, the expression of TRAC or TRBC, CIITA, and at least one NK activating receptor binding molecule of the engineered immune cell is suppressed or silenced. In a preferred embodiment, the expression of TRAC or TRBC, B2M, CIITA and at least one NK activating receptor binding molecule of the engineered immune cell is suppressed or silenced. In a preferred embodiment, the expression of TRAC or TRBC, B2M, RFX5, and at least one NK activating receptor binding molecule of the engineered immune cell is suppressed or silenced.

In an embodiment, the engineered immune cell of the present disclosure further comprises suppressed or silenced expression of one or more genes selected from the following: CD52, GR, dCK, and immune checkpoint gene, such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3. Preferably, CD52, dCK, PD1, LAG3, TIM3, CTLA4, TIGIT or a combination thereof of the engineered immune cell is suppressed or silenced.

In an embodiment, the engineered cell further expresses an immune recognition receptor, which comprises a ligand binding domain, a transmembrane domain and an intracellular signaling domain. Preferably, the immune recognition receptor is a chimeric antigen receptor or a T cell receptor. More preferably, the immune recognition receptor is a chimeric antigen receptor, which comprises a ligand binding domain, a transmembrane domain, a costimulatory domain and an intracellular signaling domain.

In an embodiment, the immune recognition receptor binds to a target selected from the group consisting of TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6/E7, MAGE A1, ETV6-AML, sperm protein 17, XAGE1, Tie 2, MAD-CT-1, MAD-CT-2, Fos-related antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D and any combination thereof. Preferably, the target is selected from the group consisting of CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin and any combination thereof.

In an embodiment, the transmembrane domain is a transmembrane domain of a protein selected from the group consisting of TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154. Preferably, the transmembrane domain is selected from transmembrane domains of CD8α, CD4, CD28 and CD278.

In an embodiment, the intracellular signaling domain is selected from the signaling domains of the following proteins: FcRγ, FcRβ, CD3γ, CD3δ, CD3ε, CD3ζ, CD22, CD79a, CD79b and CD66d. Preferably, the intracellular signaling domain is a signaling domain comprising CD3ζ.

In an embodiment, the costimulatory domain is one or more costimulatory signaling domains of a protein selected from the group consisting of: TLR1, TLR2, TLR3, TLR4, TLR5, TLR6, TLR7, TLR8, TLR9, TLR10, CARD11, CD2, CD7, CD8, CD18(LFA-1), CD27, CD28, CD30, CD40, CD54(ICAM), CD83, CD134(OX40), CD137(4-1BB), CD270(HVEM), CD272(BTLA), CD276(B7-H3), CD278(ICOS), CD357(GITR), DAP10, DAP12, LAT, NKG2C, SLP76, PD-1, LIGHT, TRIM, ZAP70 and a combination thereof. Preferably, the costimulatory domain is a costimulatory signaling domain of CD27, CD28, CD134, CD137 or CD278 or a combination thereof.

In an embodiment, the immune cell is selected from the group consisting of a T cell, a macrophage, a dendritic cell, a monocyte, an NK cell, or an NKT cell. Preferably, the immune cell is a T cell selected from the group consisting of CD4+/CD8+ T cell, CD4+ helper T cell, CD8+ T cell, tumor infiltrating cell, memory T cell, naive T cell, γδ-T cell, or αβ-T cell.

In a second aspect, the present disclosure further provides a pharmaceutical composition, comprising the engineered immune cell of the present disclosure and one or more pharmaceutically acceptable excipients.

In a third aspect, the present disclosure further provides a method of treating a subject with cancer, infection or autoimmune disease, comprising administering to the subject an effective amount of the engineered immune cell or the pharmaceutical composition according to the present disclosure. Therefore, the present disclosure further provides use of the engineered immune cell or pharmaceutical composition of the present disclosure in the preparation of a medicament for treatment of cancers, infection, or autoimmune diseases.

### Detailed Description

Unless otherwise indicated, all scientific and technical terms used herein have the same meaning as commonly understood by a person ordinarily skilled in the art to which the present disclosure belongs.

NK cells express multiple activating receptors and inhibitory receptors on the surface and its killing function is regulated by the balance of positive signals and negative signals provided by these two types of receptors. In normal situations, inhibitory receptors that recognize MHC class I molecules on cell surface dominate in signaling balance. However, when MHC class I molecules are abnormally expressed, e.g., when artificially suppressed or knocked out, corresponding activating receptors will dominate, so that the NK cells are activated, thereby exerting a cytotoxic effect and releasing cytokines. In the case of allotransplantation of engineered immune cells, such cytotoxic effect of the NK cells will kill infused therapeutic cells, thereby reducing the therapeutic effect. Therefore, it is necessary to modify the engineered immune cells, so that when the engineered immune cells are infused back to patients' body, the risk of being killed by the NK cells can be reduced or avoided, thus prolonging the survival time of therapeutic immune cells and improving the therapeutic effect.

Therefore, in a first aspect, the present disclosure provides an engineered immune cell, wherein expression of at least one MHC related gene and at least one NK activating receptor binding molecule is suppressed or silenced, so as to suppress the killing of the engineered immune cell by NK cells.

### NK activating receptor binding molecule

As used herein, the term "NK activating receptor binding molecule" refers to a molecule capable of binding to the NK activating receptor and activating the NK cell function (e.g., killing function). Non-limiting examples of NK activating receptor include NK cell surface receptors that have an immunoreceptor tyrosine-based activation motif (ITAM) or bind thereto. Such receptors can be classified into the Ig superfamily and the C-type lectin superfamily according to differences in molecular structure, including, but not limited to, NKG2C, NKG2E, NKG2D, NKp30, NKp44, NKp46, NKp80, 2B4, DNAM-1, CD2, LFA-1, etc.

In an embodiment, the NK activating receptor binding molecule is a molecule that binds to NKG2C, NKG2E or NKG2D. NKG2C, NKG2E and NKG2D all belong to the NKG2 family, and they bind to a regulatory molecule through an amino acid of the transmembrane region, further activating a downstream signaling pathway. Expression of NKG2C and NKG2E requires the formation of a heterodimer with CD94, by binding to the DAP12 molecule comprising ITAM, two tyrosine residues in ITAM are rapidly phosphorylated, further recruiting ZAP-70 and Syk, and resulting in activation of NK cells. NKG2C and NKG2E recognize the ligand HLA-E in human, and recognize the ligand Qa1b in mice. NKG2D is expressed very broadly. It is expressed not only in all NK cells but also in most γδT cells and activated CD8+ αβ T cells in human, and is expressed in all NK cells, some γδT cells and some macrophages in mice. NKG2D, expressed in a form of homodimer, can bind to two different adapter proteins DAP10 and DAP12, and mediate different functions via two signaling pathways, respectively. On one hand, NKG2D can bind to DAP10 via a positively charged arginine residue of the transmembrane region, so as to make DAP10 phosphorylated, and further activate the NK cells to exert cytotoxic effect via a PI3K-dependent Ras-independent signaling pathway. On the other hand, NKG2D can also bind to ITAM comprising DAP12, and recruit tyrosine kinases such as Syk and ZAP-70 by phosphorylation of tyrosine residues, resulting in the transduction of downstream signals, and inducing release of cytokines and chemokines. For NKG2D, the ligands in human are MIC genes (including MICA and MICB) and ULBP genes (including ULBP1, ULBP2, ULBP3, ULBP4, ULBP5 and ULBP6), and the ligands in mice are Rae-1, H60 and MULT1. MICA and MICB are located at one side of HLA-B locus in the MHC gene complex, and have homology of up to 91%. The ULBP gene is structurally similar to MHC class I molecules, and they both comprise α1 and α2 domains, but the ULBP gene does not comprise α3 functional domain or β2 microglobulin. Therefore, in an embodiment, the NK activating receptor binding molecule is selected from the group consisting of HLA-E, Qa1b, MICA, MICB, ULBP1, ULBP2, ULBP3, ULBP4, ULBP5, ULBP6, Rae-1, H60 and MULT1. More preferably, the NK activating receptor binding molecule is selected from the group consisting of MICA, MICB, ULBP1 and ULBP2.

In an embodiment, the NK activating receptor binding molecule is a molecule that binds to NKp30, NKp44, NKp46 or NKp80. NKp30, NKp44, Nkp46 and NKp80 all belong to the family of natural cytotoxicity receptors (NCR), and the expression profile thereof on the cell surface is closely related to functionalization of the NK cells. NKp30, serving as one of the key factors for the NK cells to exert the killing activity, has a dysregulated expression level in multiple tumors and virus infection, and may participate in immune escape of tumors and viruses. The extracellular segment of NKp30 is a V-type immunoglobulin-like domain, which is linked to an arginine-rich transmembrane region through a hydrophobic amino acid sequence. NKp30 binds to CD3ζ and transmits activation signals into cells via the ITAM of the latter. Activating ligands for NKp30 include B7-H6, BAG6 (also known as BAT3), PfEMP1, HSPGS, and the like. The extracellular region of NKp44 comprises a V-type domain, and the transmembrane region has charged lysines binding to KAPAP/DAP12. Although comprising ITIM in the intracellular region, NKp44 lacks a suppressing function and cannot attenuate the activation signal transmitted by DAP12. Researches show that NKp44 can bind to hemagglutinin of virus and exert the antiviral effect of the NK cells. The extracellular region of NKp46 has two type C2 Ig-like domains, the transmembrane region comprises a positively charged arginine residue, and the intracellular region does not comprise an ITAM motif, therefore, similar to NKp30, the NKp46 also needs to form a complex with a molecule, such as CD3ζ and/or FcεRIγ, comprising ITAM in the intracellular segment, to collectively transmit an activation signal into cells. NKp46 is expressed on the surface of all mature NK cells and is a key receptor for initiating the killing function of the NK cells. Nkp46 also can bind to hemagglutinin of virus and exert the antiviral effect of the NK cells. In addition, NKp46 is also capable of binding to HSPGS. NKp80 is expressed on the surface of almost all NK cells in the form of homodimer, and can rapidly activate the NK cells after binding to its ligand activation-induced C-type lectin (AICL), improving the NK cells' cytotoxicity and ability to secret inflammatory cytokines. Therefore, in an embodiment, the NK activating receptor binding molecule is selected from the group consisting of B7-H6, BAG6, PfEMP1, HSPGS and AICL, preferably from B7-H6, BAG6 and AICL.

In an embodiment, the NK activating receptor binding molecule is a molecule that binds to 2B4. 2B4, also known as CD244, is a membrane protein that is widely expressed on NK cells, CD8+ T cells, monocytes and granulocyte surfaces. The extracellular region of 2B4 has a V-type immunoglobulin domain and a C2-type immunoglobulin-like domain, the transmembrane region does not comprise any charged amino acid, the intracellular region comprises an immunoreceptor tyrosine-based inhibitory switch motif (ITSM), and the motif can be recognized by cytoplasmic SH2 region of adaptor proteins SAP, EAT-2, DRT, etc. 2B4 cross-linking makes tyrosine in ITSM phosphorylated and recruits adaptor proteins, wherein the complex formed by 2B4 and SAP can activate the NK cells. 2B4 is not an independent receptor, and as a co-activating receptor for the NK cells, its initiation depends on co-action with other NCR receptors. The ligand for 2B4 is CD48, which is highly expressed on hematopoietic cell lines and some B lymphocytes. Therefore, in an embodiment, the NK activating receptor binding molecule is CD48.

In an embodiment, the NK activating receptor binding molecule is a molecule that binds to DNAM-1. DNAM-1, also known as CD226, is a major co-activating receptor that initiates the functionality of the NK cells. DNAM-1 comprises an extracellular region containing two immunoglobulin V-like domains, one transmembrane region, and a cytoplasmic region comprising potential phosphorylation sites for tyrosine and serine residues. DNAM-1 is expressed on a variety of hemocytes, including T cells, NK cells, NKT cells, monocytes, granulocytes and platelets. DNAM-1 is a common receptor of CD155 and CD112. CD112 is a herpes simplex virus receptor, belongs to the family of human nectin, and is a Ca²⁺ independent IgSF adhesion molecule. The family members thereof interact with each other in a homologous or heterologous form, causing intercellular adhesion. CD155 is structurally similar to nectin, and is also known as nectin-like molecule 5 (NECL5). Therefore, in an embodiment, the NK activating receptor binding molecule is selected from CD112 and CD155.

In an embodiment, the NK activating receptor binding molecule is a molecule that binds to CD2. CD2, also known as LFA-2, is a single chain glycoprotein consisting of 327 amino acids and is expressed on the surface of mature T cells, most thymocytes and some NK cells. Ligands for CD2 include CD58 (also known as LFA-3) and CD59. CD58 is widely expressed on surface of non-hematopoietic cells and hematopoietic cells. By means of specific recognition with CD2 molecules, CD58 can activate phospholipase Cγ1. The latter mediates the generation of inositol triphosphate and diacylglycerol, so that the calcium ion concentration in cells is increased, and IL-2 transcription is further activated in a synergistic manner, thus promoting proliferation of T cells and secretion of cytokines. In addition, the interaction between CD58 and CD2 molecules also can promote the formation of TCR-polypeptide-MHC triplet complex, and further enhance antigen presentation. Researches show that if the expression of CD58 molecule is absent, NK cells and T cells cannot be effectively activated. CD59 is a glycoprotein with a molecular weight around 20 KD anchored to the cell membrane by GPI. CD59 binds near to Gly95 of CD2 and binds to T cells through CD2 molecules so as to participate in adhesion between T cells and target cells. Researches show that binding sites of CD58 and CD59 on CD2 are partially overlapped, and they both participate in the activation of CD2 molecules and synergistically promote the proliferation of lymphocytes. Therefore, in an embodiment, the NK activating receptor binding molecule is selected from CD58 and CD59.

In an embodiment, the NK activating receptor binding molecule is a molecule that binds to LFA-1. LFA-1 consists of two polypeptide chains linked by a non-covalent bond: α subunit (CD11a) and β subunit (CD18). Ligands for LFA-1 include the immunoglobulin superfamily members ICAM1, ICAM2 and ICAM3, which are intercellular adhesion molecules that play an important role in localizing and adhering leukocytes to epithelial cells at a site of injury. ICAM1 can be expressed on a variety of cells, e.g., lymphocytes, endothelial cells, monocytes and tumor cells, and is subjected to induced regulation of cytokine. ICAM2 can be expressed on leukocytes, endothelial cells and platelets, and ICAM3 is expressed only on leukocytes, they both are not subjected to induced regulation of cytokine. These three ligands bind to different regions within α subunit of LFA-1. The binding between ICAM molecules and LFA-1 provides necessary costimulatory signal for immune killing mediated by cytotoxic T cell and NK cell, thus activating the immune response. In the above, ICAM-1 is expressed in a form of dimer, and has the highest affinity to LFA-1. Researches show that ICAM-1 can enhance NKG2D-mediated killing effect of NK cells on colorectal cancer cells. Therefore, in an embodiment, the NK activating receptor binding molecule is selected from ICAM1, ICAM2 and ICAM3.

In an embodiment, the NK activating receptor binding molecule is a molecule related to the IFN γ signaling pathway. IFN γ plays an important role in the initiation and effector phases of immune responses, including, for example, macrophage activation, NK cell activation, B cell and T cell differentiation, upregulation of MHC class I and MHC class II molecule expression in various cell types. The IFN γ receptor comprises two subunits: α (IFNGR1) and β (IFNGR2), both belonging to the class II cytokine receptor family, and each comprising two extracellular fibronectin type III domains. The intracellular region of IFNGR1 comprises a domain that binds to kinase JAK1 and the signal transduction and transcriptional regulator STAT1, while the intracellular region of IFNGR2 comprises a region that binds to kinase JAK2 for participating in signal transduction. In the killing process of the NK cells, a large amount of IFN γ is released, and after the IFN γ binds to IFN γ R on surface of tumor cells, JAK-STAT pathway is activated, so that the NK cells are activated and its killing against the tumor cells is further induced. Therefore, in an embodiment, the NK activating receptor binding molecule is selected from STAT1, JAK1, IFNGR2, JAK2 and IFNGR1.

Therefore, in an embodiment, the NK activating receptor binding molecule binds to an NK activating receptor selected from the group consisting of NKG2C, NKG2E, NKG2D, NKp30, NKp44, NKp46, NKp80, 2B4, DNAM-1, CD2 and LFA-1. Preferably, the NK activating receptor binding molecule binds to an NK activating receptor selected from the group consisting of NKG2D, Nkp30, 2B4, DNAM-1, CD2 and LFA-1. In another embodiment, the NK activating receptor binding molecule is a molecule related to the IFNγ signaling pathway. Therefore, in an embodiment, the NK activating receptor binding molecule is selected from the group consisting of MICA, MICB, ULBP1, ULBP2, ULBP3, ULBP4, ULBP5, ULBP6, Rae-1, H60, MULT1, B7-H6, BAG6, PfEMP1, HSPGS, AICL, CD112, CD155, CD48, CD58, CD59, ICAM1, ICAM2, ICAM3, STAT1, JAK1, IFNGR2, JAK2 and IFNGR1. Preferably, the NK activating receptor binding molecule is selected from the group consisting of CD112, CD155, CD48, MICA, MICB, CD58, B7-H6, ICAM1, ICAM2 and ICAM3.

In an embodiment, the NK activating receptor binding molecule further may be other known NK activating ligands, including, but not limited to, for example, CD111, CD113, CEACAM1, CEACAM7, SMAP2, IL23R, PCGF5, DET1, PTGFRN, CD84, CADM1, CD72, CD74, BTN3A3, CD47, CTSS, NTRK2, RTP4, TLR3/CD283, TMEM140, TMPRSS3, BST2/CD317, BTN3A1, CD40, EPST11, ERAP1, ERAP2, GJD3, HCP5, IFI6, IFITM1, IFITM2, IFITM3, LGALS3BP, C1QBP, CD24, CD55, CD9, GJA1, GPRC5B, HMMR, IGSF5, SYNGR3, TFRC/CD71, CD90, TMEM68, TMEM97 and ANKRD27.

### MHC related gene

The major histocompatibility complex (MHC) is originally characterized as protein that plays a major role in the transplantation reaction, and it is expressed on surface of all higher vertebrates. It is called as H-2 in mice, and HLA in human cells. MHC mainly has two types: class I, and class II. MHC class I protein is a heterodimer of two proteins: one is the transmembrane protein α chain encoded by MHCI gene, and the other is the β2 microglobulin chain of an extracellular protein encoded by a gene not located in the MHC gene cluster. The α chain includes three domains, and a foreign peptide binds to two most variable domains α1, α2 at N terminal. MHC class II protein is also a heterodimer, and comprises two transmembrane proteins encoded by genes within the MHC complex. MHC class I/antigen complex interacts with cytotoxic T cells, whereas MHC class II presents antigen to helper T cells. In addition, MHC class I protein tends to be expressed in almost all nucleated cells and platelets (as well as red blood cells in mice), while MHC class II protein is more selectively expressed. Generally, MHC class II protein is expressed on B cells, some macrophages and monocytes, Langerhans cells and dendritic cells.

The human HLA class I gene cluster comprises three major loci B, C and A. The class I molecule is a heterodimer consisting of MHC α heavy chain (encoded by HLA-A, HLA-B or HLA-C) and light chain (β-2 microglobulin, encoded by B2M). The heavy chain is anchored in the membrane. It is about 45 kDa, and comprises 8 exons. Exon 1 encodes a leader peptide, exons 2 and 3 encode α1 and α2 domains, both of which bind to peptides, exon 4 encodes α3 domain, exon 5 encodes a transmembrane region, and exon 6 and 7 encode cytoplasmic tail. Polymorphism within exon 2 and exon 3 results in peptide binding specificity for each class of molecules. Thus, in an embodiment, suppressing or silencing the expression of MHC related gene refers to suppressing or silencing the expression of one or more genes selected from the group consisting of HLA-A, HLA-B, HLA-C and B2M.

Human HLA class II cluster also comprises three major loci DP, DQ and DR, and both class I gene cluster and class II gene cluster are polymorphic. The class II molecule plays a major role in the immune system by presenting exogenous peptides, and is primarily expressed in antigen presenting cells (e.g., B lymphocyte, dendritic cell and macrophage). Class II molecule is a heterodimer consisting of α chain and β chain both anchored in the membrane, wherein the α chain is about 33-35 kDa, and comprises 5 exons. Exon 1 encodes a leader peptide, exons 2 and 3 encode two extracellular domains, exon 4 encodes a transmembrane domain, and exon 5 encodes a cytoplasmic tail. Thus, in an embodiment, suppressing or silencing the expression of the MHC related gene refers to suppressing or silencing the expression of one or more genes selected from the group consisting of HLA-DPA, HLA-DQ and HLA-DRA.

Expression of MHC class I and class II also depends on a variety of helper proteins. For example, Tap1 and Tap2 subunits are part of TAP transporter complex necessary for loading peptide antigens on the HLA class I complex. LMP2 and LMP7 proteosome subunits function in proteolytic degradation of antigens to peptides so as to be displayed on HLA. Reducing LMP7 has been shown to reduce the expression level of MHC class I at the cell surface. MHC class II expression is induced and expressed by some positive regulators, for example, RFX complex and CIITA. The RFX complex consists of three subunits: RFXANK (also known as RFXB), RFX5 and RFX accessory protein (also known as RFXAP). The RFX complex promotes the expression of MHC class II molecules by promoting the binding of other transcription factors to the promoter of MHC class II molecules and enhancing the specificity of the promoter binding. CIITA is a major control factor for MHC class II expression. CIITA comprises an N terminal rich in acidic amino acids, a PST region rich in Pro, Ser and Thr, an intermediate GTP binding domain, and a C terminal rich in Leu repeat sequence (LRR), wherein the N terminal acidic region and the PST region are transcriptional activation regions. Thus, in an embodiment, suppressing or silencing the expression of MHC related gene refers to suppressing or silencing the expression of one or more genes selected from the group consisting of TAP1, TAP2, LMP2, LMP7, RFX5, RFXAP, RFXANK and CIITA.

Therefore, in an embodiment, the MHC related gene is selected from the group consisting of MHC class I molecules, e.g., HLA-A, HLA-B, HLA-C and B2M; MHC class II molecules, e.g., HLA-DPA, HLA-DQ and HLA-DRA; and MHC helper proteins, e.g., TAP1, TAP2, LMP2, LMP7, RFX5, RFXAP, RFXANK and CIITA, preferably from HLA-A, HLA-B, HLA-C, B2M, RFX5, RFXAP, RFXANK, CIITA and a combination thereof. In another preferred embodiment, the MHC related gene is selected from MHC class II molecules and MHC accessory proteins, e.g., HLA-DPA, HLA-DQ, HLA-DRA, TAP1, TAP2, LMP2, LMP7, RFX5, RFXAP, RFXANK and CIITA.

### Engineered immune cell

As used herein, the term "immune cell" refers to any cell of the immune system that has one or more effector functions (e.g., cytotoxic cell killing activity, secretion of cytokines, and induction of ADCC and/or CDC). For example, the immune cell may be a T cell, a macrophage, a dendritic cell, a monocyte, an NK cell or an NKT cell, or an immune cell derived from stem cells (e.g., adult stem cells, embryonic stem cells, umbilical cord blood stem cells, progenitor cells, bone marrow stem cells, induced pluripotent stem cells, totipotent stem cells, or hematopoietic stem cells). Preferably, the immune cell is a T cell. The T cell may be any T cell, such as *in vitro* cultured T cell, for example, primary T cell or T cell from *in vitro* cultured T cell line, e.g., Jurkat and SupT1, or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from infection sites, ascites, pleural effusion, spleen tissue and tumors. The T cell also may be concentrated or purified. The T cell may be at any developmental stage including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell, etc. In a preferred embodiment, the immune cell is a human T cell. The T cell can be isolated from the blood of a subject by using a variety of techniques known to a person skilled in the art, such as Ficoll.

When the engineered immune cell of the present disclosure expresses an immune recognition receptor (e.g., a chimeric antigen receptor or a T cell receptor), the immune recognition receptor can be introduced into the immune cells by a conventional method known in the art (e.g., by transduction, transfection, transformation, etc.). "Transfection" is a process of introducing a nucleic acid molecule or polynucleotide (including a vector) into a target cell. An example is RNA transfection, i.e., a process of introducing RNA (such as *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out by using calcium phosphate, by electroporation, by extrusion of cells or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat-shock-mediated uptake, fusion to bacterial protoplasts of intact cells, microinjection and electroporation.

In an embodiment, in addition to suppressing or silencing expression of at least one MHC related gene and at least one NK activating receptor binding molecule, expression of at least one TCR/CD3 gene of the engineered immune cell of the present disclosure further may be suppressed or silenced.

T cell receptor (TCR) is a characteristic marker of the surface of all T cells. It forms a TCR/CD3 complex by binding to CD3 through a non-covalent bond, and generates a specific antigen stimulation signal by binding to a specific MHC-antigen peptide complex on the surface of the antigen presenting cell, thus to activate T cell and exert the killing effect. TCR is a heterodimer composed of two different peptide chains, and is generally divided into two types: α/β type and γ/δ type, in which 95% or more of peripheral T lymphocytes express TCR α/β. The TCR α chain is encoded by the TRAC gene, and the β chain is encoded by the TRBC gene. Each peptide chain of the TCR includes a variable region (V region), a constant region (C region), a transmembrane region, and a cytoplasmic region, wherein the cytoplasmic region is very short, and does not have the capability of delivering an antigen stimulation signal. TCR molecule belongs to the immunoglobulin superfamily, and the antigen specificity thereof exists in V region; the V region has three hypervariable regions, i.e., CDR1, CDR2 and CDR3, wherein the CDR3 has the maximum variation, and directly determines the antigen binding specificity of the TCR. When TCR recognizes MHC-antigen peptide complexes, CDR1 and CDR2 recognize and bind to the MHC molecule, and CDR3 directly binds to the antigen peptide. CD3 includes four subunits: γ, δ, ε, ζ, usually existing in the form of dimers εγ, εδ and ζζ. All of the four subunits comprise a conserved immunoreceptor tyrosine-based activation motif (ITAM), in which two tyrosine residues, after being phosphorylated by tyrosine protein kinases, transmit an activation signal to T cells. Therefore, in an embodiment, the TCR/CD3 gene is selected from the group consisting of TRAC, TRBC, CD3 γ, CD3 δ, CD3 ε, CD3 ζ and a combination thereof.

In a preferred embodiment, expression of at least one MHC related gene, at least one NK activating receptor binding molecule, and at least one TCR/CD3 gene of the engineered immune cell of the present disclosure is suppressed or silenced, wherein definitions of MHC related gene, NK activating receptor binding molecule, and TCR/CD3 gene are as mentioned above. In a preferred embodiment, expression of at least one MHC related gene, at least one NK activating receptor binding molecule, and at least one TCR/CD3 gene of the engineered immune cell of the present disclosure is suppressed or silenced, wherein the MHC related gene is selected from the group consisting of HLA-A, HLA-B, HLA-C, B2M, RFX5, RFXAP, RFXANK, CIITA and a combination thereof, the NK activating receptor binding molecule is selected from the group consisting of CD112, CD155, CD48, MICA, MICB, CD58, B7-H6, ICAM1, ICAM2, ICAM3 and a combination thereof, and the TCR/CD3 gene is selected from the group consisting TRAC, TRBC, CD3γ, CD3δ, CD3ε, CD3ζ and a combination thereof.

In an embodiment, expression of at least one gene selected from the following in the engineered immune cell of the present disclosure further may be suppressed or silenced: CD52, GR, dCK and immune checkpoint genes, such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3.

Methods for suppressing gene expression or silencing a gene are well known to a person skilled in the art. For example, expression of a gene can be suppressed by using antisense RNA, RNA decoy, RNA aptamer, siRNA, shRNA, miRNA, trans-dominant negative protein (TNP), chimeric/fusion protein, chemokine ligand, anti-infective cellular protein, intracellular antibodies (sFvs), nucleoside analogs (NRTI), non-nucleoside analogs (NNRTI), integrase inhibitors (oligonucleotides, dinucleotides and chemical agents) and protease inhibitors. In addition, for example, a meganuclease, a zincfinger nuclease, a TALE nuclease, or a Cas enzyme in a CRISPR system can also be used to mediate DNA breakage, thereby silencing the gene.

In an embodiment, the engineered cell further expresses an immune recognition receptor, which comprises a ligand binding domain, a transmembrane domain and an intracellular signaling domain. Preferably, the immune recognition receptor is a chimeric antigen receptor or a T cell receptor. More preferably, the immune recognition receptor is a chimeric antigen receptor, which comprises a ligand binding domain, a transmembrane domain, a costimulatory domain and an intracellular signaling domain.

Definition of the T cell receptor or TCR is as described in the above. In the present disclosure, the TCR is a recombinant TCR, which may comprise a heterologous ligand-binding domain (e.g., an antibody).

As used herein, the term "chimeric antigen receptor" or "CAR" refers to an artificially constructed hybrid polypeptide, where the hybrid polypeptide generally includes one or more ligand binding domains (e.g., an antigen binding moiety of an antibody), a transmembrane domain, a costimulatory domain and an intracellular signaling domain, and various domains are linked via a linker. CAR can redirect the specificity and reactivity of T cells and other immune cells to selected targets in a non-MHC-restricted manner by utilizing the antigen binding properties of monoclonal antibodies. Non-MHC-restricted antigen recognition gives CAR cells the ability to recognize an antigen independent of antigen processing, thus bypassing the major mechanism of tumor escape. Furthermore, when expressed within T cells, CAR advantageously does not dimerize with α chain and β chain of the endogenous T cell receptor (TCR).

As used herein, "ligand binding domain" refers to any structure or functional variant thereof that can bind to a ligand (e.g., antigen). The ligand binding domain may be an antibody structure, including, but not limited to, monoclonal antibody, polyclonal antibody, recombinant antibody, human antibody, humanized antibody, murine antibody, chimeric antibody and functional fragments thereof. For example, the ligand binding domain includes, but is not limited to, intact antibody, Fab, Fab', F(ab')2, Fv fragment, scFv antibody fragment, linear antibody, sdAb (VH or VL), nanobody (Nb), recombinant fibronectin domain, anticalin, DARPIN, and so on, preferably from Fab, scFv, sdAb and nanobody. In the present disclosure, the ligand binding domain may be monovalent or bivalent, and may be a monospecific, bispecific or multispecific antibody

"Fab" refers to any one of two identical fragments produced after an immunoglobulin molecule is cleaved by papain, and consists of an intact light chain and a heavy chain N terminal part linked by a disulfide bond, wherein the heavy chain N terminal part includes a heavy chain variable region and CH1. Compared with intact IgG, Fab has no Fc fragment, has relatively high fluidity and tissue penetration ability, and can univalently bind to an antigen without mediating antibody effects.

"Single chain antibody" or "scFv" is an antibody composed of antibody's heavy chain variable region (VH) and light chain variable region (VL) linked by a linker. The optimal length and/or amino acid composition of the linker can be selected. The length of the linker will significantly affect the variable region folding and interaction of the scFv. In fact, intrachain folding can be prevented if a shorter linker (e.g., 5-10 amino acids) is used. Regarding the selection of the size and composition of the linker, see, e.g., Hollinger et al., 1993 Proc Natl Acad. Sci. U.S.A. 90:6444-6448; US patent application with publication numbers 2005/0100543, 2005/0175606, 2007/0014794; and PCT application WO2006/020258 and WO2007/024715, which are herein incorporated by reference in their entirety. The scFv may comprise a VH and a VL linked in any order, e.g., VH-linker-VL or VL-linker-VH.

"Single domain antibody" or "sdAb" refers to an antibody that naturally lacks a light chain, and this antibody comprises only one heavy chain variable region (VHH) and two conventional CH2 and CH3 regions, also known as "heavy chain antibody".

"Nanobody" or "Nb" refers to a VHH structure that is individually cloned and expressed, which has structural stability and binding activity to an antigen comparable to those of the original heavy chain antibody and binding activity to an antigen, and is the smallest unit currently known to be capable of binding to a target antigen.

The term "functional variant" or "functional fragment" refers to a variant that substantially comprises the amino acid sequence of a parent, but, compared with the parent amino acid sequence, comprises at least one amino acid modification (i.e., substitution, deletion, or insertion), provided that the variant retains the biological activity of the parent amino acid sequence. For example, for an antibody, a functional fragment thereof is an antigen binding portion thereof. In an embodiment, the amino acid modification is preferably a conservative modification.

As used herein, the term "conservative modification" refers to amino acid modification that does not obviously affect or alter the binding characteristics of the antibody or antibody fragment comprising the amino acid sequence. These conservative modifications include amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is a substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine) and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with completely identical sequences have 100% identity. A person skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197) and ClustalW.

The selection of ligand binding domain depends on the cell surface marker on a target cell to be recognized and associated with a specific disease state, for example, a tumor specific antigen or a tumor associated antigen. Thus, in an embodiment, the ligand binding domain of the present disclosure binds to one or more targets selected from the group consisting of TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAIX, LMP2, gp100, bcr-ab1, tyrosinase, EphA2, Fucosyl, GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos-related antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, Claudin18.2, NKG2D and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAlX, WT1, NY-ESO-1, CD79a, CD79b, GPC3, Claudin18.2, NKG2D and any combination thereof. Depending on the antigen to be targeted, the CAR of the present disclosure may be designed to include a ligand binding domain specific for the antigen. For example, if CD19 is the antigen to be targeted, a CD19 antibody can be used as the ligand binding domain of the present disclosure.

As used herein, the term "transmembrane domain" refers to a polypeptide structure that enables expression of a chimeric antigen receptor on the surface of an immune cell (e.g., a lymphocyte, an NK cell or an NKT cell), and guides the cellular response of the immune cell against the target cell. The transmembrane domain may be natural or synthetic, and also may be derived from any membrane-bound protein or transmembrane protein. When the chimeric antigen receptor binds to the target antigen, the transmembrane domain is capable of signaling. The transmembrane domains particularly suitable for use in the present disclosure may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CD8 α, CD9, CD16, CD22, CD33, CD28, CD37, CD64, CD80, CD86, CD134, CD137, CD154 and functional fragments thereof. Alternatively, the transmembrane domain may be synthesized and may mainly comprise a hydrophobic residue such as leucine and valine. Preferably, the transmembrane domain is derived from a CD8 α chain.

In an embodiment, the chimeric antigen receptor of the present disclosure further may comprise a hinge region located between the ligand binding domain and the transmembrane domain. As used herein, the term "hinge region" generally refers to any oligopeptide or polypeptide that functions to link a transmembrane domain to a ligand binding domain. Specifically, the hinge region serves to provide greater flexibility and accessibility to the ligand binding domain. The hinge region may comprise up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence or may be a completely synthetic hinge sequence. In a preferred embodiment, the hinge region comprises a CD8 α chain, an Fc γ RIII α receptor, a hinge region portion of IgG4 or IgG1, more preferably a CD8 a hinge.

As used herein, the term "intracellular signaling domain" refers to a protein fraction that transduces an effector function signal and guides a cell to perform a specified function. The intracellular signaling domain is responsible for intracellular primary signaling after the ligand binding domain binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, including secretion of cytokines.

In an embodiment, the intracellular signaling domain comprised in the chimeric antigen receptor of the present disclosure may be cytoplasmic sequences of a T cell receptor and a co-receptor, and upon antigen receptor binding, which act together to initiate primary signaling, as well as any derivative or variant of these sequences and any synthetic sequence having the same or similar function.

The intracellular signaling domain may contain many immunoreceptor tyrosine-based activation motifs (ITAM). Non-limiting examples of intracellular signaling domains of the present disclosure include, but are not limited to, those derived from FcR γ, FcR β, CD3 γ, CD3 δ, CD3 ε, CD3 ζ, CD22, CD79a, CD79b, and CD66d. In a preferred embodiment, the signaling domain of the CAR of the present disclosure may comprise a CD3 ζ signaling domain.

In an embodiment, the chimeric antigen receptor of the present disclosure comprises one or more costimulatory domains. The costimulatory domain may be an intracellular functional signaling domain from a costimulatory molecule, and it comprises an entire intracellular portion of the costimulatory molecule or a functional fragment thereof. "Costimulatory molecule" refers to a homologous binding partner that specifically binds to a costimulatory ligand on a T cell, thereby mediating a costimulatory response (e.g., proliferation) of the T cell. The costimulatory molecule includes, but is not limited to, MHC class I molecules, BTLA, and Toll ligand receptors. Non-limiting examples of the costimulatory domain of the present disclosure include, but are not limited to, costimulatory signaling domains derived from a protein selected from the group consisting of TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA) , CD276 (B7-H3), CD278 (ICOS), CD357 (GITR), DAP10, DAP12, LAT, NKG2C, SLP76, PD-1, LIGHT, TRIM, and ZAP70. Preferably, the costimulatory domain of the CAR of the present disclosure is from 4-1BB, CD28, CD27, OX40, or a combination thereof. In an embodiment, the CAR of the present disclosure comprises 4-1BB, CD28 or 4-1BB+CD28 costimulatory domain.

In an embodiment, the CAR of the present disclosure further may comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to a person skilled in the art, for example, signal peptides derived from CD8 α, IgG1, and GM-CSFRα.

In an embodiment, the CAR of the present disclosure further may comprise a switch structure, so as to regulate expression time of the CAR. For example, the switch structure may be in the form of a dimerization domain, which causes a conformational change by binding to a corresponding ligand thereof, and exposes the extracellular binding domain to enable its binding a targeted antigen, thereby activating a signaling pathway. Alternatively, a switch domain also may be used to link the binding domain and the signaling domain, respectively, and only when the switch domains are bound to each other (for example, in the presence of an inducing compound), the binding domain and the signaling domain can be linked together by a dimer, thereby activating the signaling pathway. The switch structure further can be in the form of a masking peptide. The masking peptide can shield the extracellular binding domain, and prevent it from binding to the targeted antigen, and when the masking peptide is cleaved by, for example, a protease, the extracellular binding domain is exposed, making it become a "normal" CAR structure. A variety of switch structures known to a person skilled in the art can be used in the present disclosure.

In an embodiment, the CAR of the present disclosure further may comprise a suicide gene, to make it express a cell death signal that can be induced by an exogenous substance, so as to eliminate the CAR cell when needed (e.g., when serious toxic side effects are produced). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce the cell to die when receiving ganciclovir treatment. The suicide gene further may be iCaspase-9, and dimerization of iCaspase-9 can be induced by a chemical induction drug such as AP1903 and AP20187, so as to activate a downstream Caspase3 molecule, and cause apoptosis. A variety of suicide genes known to a person skilled in the art can be used in the present disclosure.

### Pharmaceutical composition

The present disclosure further provides a pharmaceutical composition, which comprises the engineered immune cell of the present disclosure as an active agent and a pharmaceutically acceptable excipient. Therefore, the present disclosure further encompasses use of the engineered immune cell in the preparation of a pharmaceutical composition or a medicine.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania Mack Publishing Company, 1995). Examples of the pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to a person skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition comprising, for example, the immune cell as described herein is generally provided in a form of solution, and preferably comprises a pharmaceutically acceptable buffer.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic use

The present disclosure further provides a method of treating a subject with cancer, infection or autoimmune disease, including administering to the subject an effective amount of the engineered immune cell or the pharmaceutical composition according to the present disclosure. Therefore, the present disclosure also encompasses use of the engineered immune cell or pharmaceutical composition in the preparation of a medicine for treatment of cancers, infection, or autoimmune diseases.

In an embodiment, an effective amount of the immune cell and/or the pharmaceutical composition of the present disclosure is directly administered to the subject.

In another embodiment, the treatment method of the present disclosure is *ex vivo* treatment. Specifically, the method includes the steps of: (a) providing a sample, the sample containing an immune cell; (b) suppressing or silencing the expression of at least one NK activating receptor binding molecule and at least one MHC related gene (and optional TCR/CD3 gene and/or other genes) of the immune cell *in vitro,* and introducing an optional immune recognition receptor (preferably chimeric antigen receptor) into the immune cell to obtain a modified immune cell, and (c) administering the modified immune cell to the subject in need thereof. Preferably, the immune cell provided in step (a) is selected from a macrophage, a dendritic cell, a monocyte, a T cell, an NK cell or an NKT cell; and the immune cell can be obtained from a sample (particularly a blood sample) of the subject by conventional methods known in the art. Besides, the immune cells generally selected are compatible with the subject's immune system, i.e., it is preferred that the immune cells do not trigger an immunogenic response. For example, a "general recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an allogeneic cell, preferably a T cell, a macrophage, a dendritic cell, a monocyte, an NK cell, or an NKT cell, more preferably a T cell, an NK cell or an NKT cell.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic materials from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse or a cow. Mammals other than human can be advantageously used as subjects representing cancer animal models. Preferably, the subject is a human.

In an embodiment, the cancer is a cancer associated with expression of the target to which the ligand binding domain binds. For example, the cancer includes, but is not limited to, brain glioma, blastoma, sarcoma, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (such as small cell lung cancer, non-small cell lung cancer, lung adenocarcinoma and squamous lung cancer), melanoma, myeloma, neuroblastoma, oral cancer (e.g., lips, tongue, mouth, and pharynx), ovarian cancer, pancreatic cancer, prostate cancer, mesothelioma, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer, Waldenstrom macroglobulinemia, lymphoma (including Hodgkin's lymphoma and non-Hodgkin's lymphoma, for example, B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, Burkitt lymphoma, diffuse large B cell lymphoma, follicular lymphoma, MALT lymphoma, marginal zone lymphoma, plasmablastic lymphoma, plasmacytoid dendritic cell tumor, etc.), leukemia (including acute leukemia, for example, acute lymphoblastic leukemia, acute myeloid leukemia, acute non-lymphocytic leukemia such as acute myeloblastic leukemia (including undifferentiated and differentiated), acute promyelocytic leukemia, acute myelo-monocytic leukemia, acute monocytic leukemia, erythroleukemia, acute megakaryoblastic leukemia; chronic leukemia, for example, chronic myelogenous leukemia, chronic lymphocytic leukemia, and chronic myelomonocytic leukemia; and other special types of leukemia, for example, hair cell leukemia, prolymphocytic leukemia, plasma cell leukemia, adult T-cell leukemia, eosinophilic leukemia, and basophilic leukemia), blastic plasmacytoid dendritic cell neoplasm, malignant lymphoproliferative disorder, bone marrow hypoplasia, multiple myeloma, bone marrow hyperplasia abnormality, post-transplant lymphoproliferative disorder (PTLD), and other diseases associated with target expression. Preferably, the disease which can be treated with the engineered immune cell or the pharmaceutical composition of the present disclosure is selected from the group consisting of: leukemia, lymphoma, multiple myeloma, myelodysplasia, brain glioma, pancreatic cancer, ovarian cancer, mesothelioma, breast cancer, lung cancer, prostate cancer, melanoma, myeloma, sarcoma, stomach cancer, and so on.

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

In an embodiment, the method further includes administering to the subject one or more additional chemotherapeutic agents, biological agents, medicines or treatments. In this embodiment, the chemotherapeutic agents, the biological agents, the medicines or the treatments are selected from the group consisting of radiotherapy, surgery, antibody reagent and/or small molecule and any combination thereof.

The present disclosure will be described in detail below with reference to the accompanying drawings and examples. It should be noted that a person skilled in the art should understand that the accompanying drawings of the present disclosure and examples thereof are only for illustrative purpose, and cannot constitute any limitation to the present disclosure. The examples of the present disclosure and the features in the examples may be combined with each other without contradiction.

### Brief Description of Drawings

FIG. 1. Expression levels of NK activating receptor binding molecules on surface of T cells.
FIG. 2. Suppression effect of DKO-T cells of the present disclosure on killing effect of NK cells.
FIG. 3. Suppression effect of QKO-T cells of the present disclosure on the killing effect of NK cells.
FIG. 4. scFv expression levels of CAR-dKO and CAR-sKO T cells of the present disclosure.
FIG. 5. Killing effects of CAR-dKO and CAR-sKO T cells of the present disclosure on target cells.
FIG. 6: Cytokine release levels of CAR-dKO and CAR-sKO T cells of the present disclosure after co-culture with target cells.

### Detailed Description of Embodiments

T cells used in all the examples of the present disclosure are primary human CD4+CD8+ T cells isolated from healthy donors by Ficoll-PaqueTM PREMIUM (GE Healthcare, Lot No. 17-5442-02) using leukapheresis.

### Example 1. Expression levels of NK activating receptor binding molecules

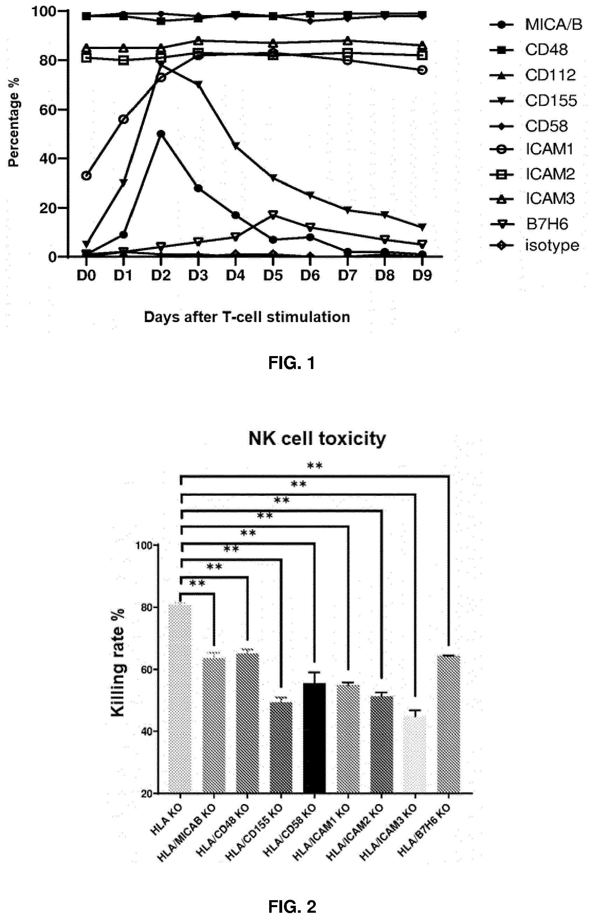

T cells were activated with DynaBeads CD3/CD28 CTSTM (Gibco, Lot No. 40203D), and cultured at 37 °C and 5% CO₂. T cells were collected at different culture time points, and expression levels of various NK activating receptor binding molecules were detected by flow cytometry, with results being shown in FIG. 1.

As can be seen from FIG. 1, the NK activating receptor binding molecules MICA/B, CD48, CD112, CD155, ICAM1, ICAM2, ICAM3, B7-H6 and CD58 show constitutive or inducible expression on T cells.

### Example 2. Construction of DKO-T cells with MHC related gene and NK activating receptor binding molecule therein being knocked out

T cells were activated with DynaBeads CD3/CD28 CTSTM (Gibco, Lot No. 40203D), and continuously cultured for 3 days at 37 °C and 5% CO₂. Then 10 µg of Cas9 protein, 5 µg of B2M sgRNA, and 5 µg of sgRNA targeting NK activating receptor binding molecule were electrically transfected into activated T cells at 400 V and 0.7 ms using a BTX Agile Pulse Max electroporator (Harvard Apparatus BTX) to obtain DKO-T cells with B2M and NK activating receptor binding molecules therein being both knocked out. T cells with only B2M being knocked out (KO-T) and wild-type T cells without knockout (NT) served as controls. The sgRNA sequences used in the present disclosure are shown in Table 1.

After DKO-T, B2M-KO-T and NT cells were cultured at 37 ºC and 5% CO₂ for 11 days, gene knockout efficiencies of B2M and various NK activating receptor binding molecules were examined by flow cytometry using corresponding antibodies (see Table 2), whose results are shown in Table 3.

**Table 1. sgRNA Sequences Used in the Present Disclosure**

| Gene name | SEQ ID NO | Sequence |
|---|---|---|
| TRAC | 1 | agagucucucagcugguaca |
| B2m | 2 | ggguagcgcgagcacagcua |
| RFX5 | 3 | gggguugcggauccaccuau |
| CD155 | 4 | ggaaaggugaggaacucccu |
| MICA/B | 5 | ggcugcccacugucccug |
| CD48 | 6 | ggacuugguacauaugaccg |
| CD58 | 7 | gggcauuacaacagccaucg |
| B7H6 | 8 | gggcaugccguaccacacac |
| ICAM1 | 9 | gguaccucuauaaccgccag |
| ICAM2 | 10 | gguggucaucauagucacgg |
| ICAM3 | 11 | ggugagggugagcuaacacg |

| | | |
|---|---|---|
| Note: in view of high homology of MICA and MICB genes, two MICA and MICB genes both can be simultaneously knocked out by the sgRNA designed in the present example. | | |

**Table 2. Information on Antibodies Used in Flow Cytometry Detection**

| Antibody name | Manufacturer | Lot No. |
|---|---|---|
| PE anti-human CD112 Antibody | biolegend | 337409 |
| APC anti-human CD155 Antibody | biolegend | 337618 |
| PE anti-human MICA/MICB Antibody | biolegend | 320906 |
| PE anti-human CD48 Antibody | biolegend | 336708 |
| PE anti-human CD58 Antibody | sino biological | 12409-MM05-P |
| Alexa FlourR 647 anti-human B7-H6 | BD | 566733 |
| APC anti-human CD54(ICAM-1)Antibody | biolegend | 353111 |
| PE anti-human CD102(ICAM-2)Antibody | biolegend | 328505 |
| APC anti-human CD50(ICAM-3) Antibody | biolegend | 330011 |

**Table 3. Gene Knockout Efficiencies in DKO-T cells**

| Cell name | Gene knocked out | NT | Expression level after knockout | Cell name | Gene knocked out | NT | Expression level after knockout |
|---|---|---|---|---|---|---|---|
| B2M KO | B2M | 99.90% | 13.10% | B2M/ICAM1 DKO | B2M | 99.90% | 17.60% |
| | | | | | ICAM1 | 76.00% | 8.20% |
| B2M/MICAB DKO | B2M | 99.90% | 14.40% | B2M/KAM2 DKO | B2M | 99.90% | 12.50% |
| | MICA/B | 69.6 | 22.5% | | ICAM2 | 99.00% | 23.90% |
| B2M/CD48 DKO | B2M | 99.90% | 17.10% | B2M/ICAM3 DKO | 82M | 99.90% | 14,10% |
| | CD48 | 99.20% | 16,4% | | ICAM3 | 99.40% | 19.60% |
| B2M/CD155 DKO | B2M | 99.90% | 14.80% | B2M/B7H6 DKO | B2M | 99.90% | 19.90% |
| | CD155 | 84% | 37.8% | | B7H6 | 46.60% | 12.3% |
| B2M/CD58 DKO | B2M | 99.90% | 17.60% | | | | |
| | CD58 | 99.60% | 18.20% | | | | |

### Example 3. Suppression effect of DKO-T cells of the present disclosure on killing effect of NK cells

The suppression effect of DKO-T cells prepared in the present disclosure on the killing effect of the NK cells was detected according to the following method: KO-T cells and DKO-T cells prepared in the present disclosure were labeled with Far-Red (invitrogen, Lot No. C34564). The labeled DKO-T cells and KO-T cells were then plated into a 96-well plate at a concentration of 1×10⁴ cells per well, and were co-cultured with addition of NK92 cells at an effector-target ratio of 2:1. After 16-18 hours, the ratio of T cells in the culture was detected by a flow cytometer, and the killing rate of the NK cells was calculated, with results being shown in FIG. 2.

It can be seen that in the case of knocking out only HLA (e.g., B2M), the killing rate of NK cells against T cells reaches 80%. Compared with this, further knocking out the NK activating receptor binding molecule (i.e., DKO-T cells of the present disclosure) can significantly suppress such killing effect.

### Example 4. Construction of QKO-T cells with TCR, MHC related gene and NK activating receptor binding molecule therein being knocked out

T cells were activated with DynaBeads CD3/CD28 CTSTM (Gibco, Lot No. 40203D), and continuously cultured for 3 days at 37 °C and 5% CO₂. Then 10 µg of Cas9 protein, 2.5 µg of B2M sgRNA, 2.5 µg of TRAC sgRNA, 2.5 µg of RFX5 sgRNA, and 2.5 µg of CD155 sgRNA were electrically transfected into activated T cells at 400 V and 0.7 ms using a BTX Agile Pulse Max electroporator (Harvard Apparatus BTX) to obtain QKO-T cells with B2M, TCR, RFX5 and CD155 therein being knocked out. KO-T cells with only B2M therein being knocked out and DKO-T cells with B2M/CD155 therein being both knockout were obtained by the same method. Gene knockout efficiencies in these cells were detected by flow cytometry, with results being as shown in Table 4.

**Table 4. Gene knockout efficiencies in QKO-T cells**

| Cell name | CD155 expression level | CD3 expression level | HLA-I expression level | HLA-II expression level |
|---|---|---|---|---|
| QKO-T | 37.50% | 5.60% | 14.30% | 20.60%, |
| DKO-T | 33.20% | 98.00% | 12.7.00% | 73.00% |
| KO-T | 88.00% | 98.00% | 12.00% | 73.00% |
| NT | 89.00% | 98.60% | 99.00% | 75.30% |

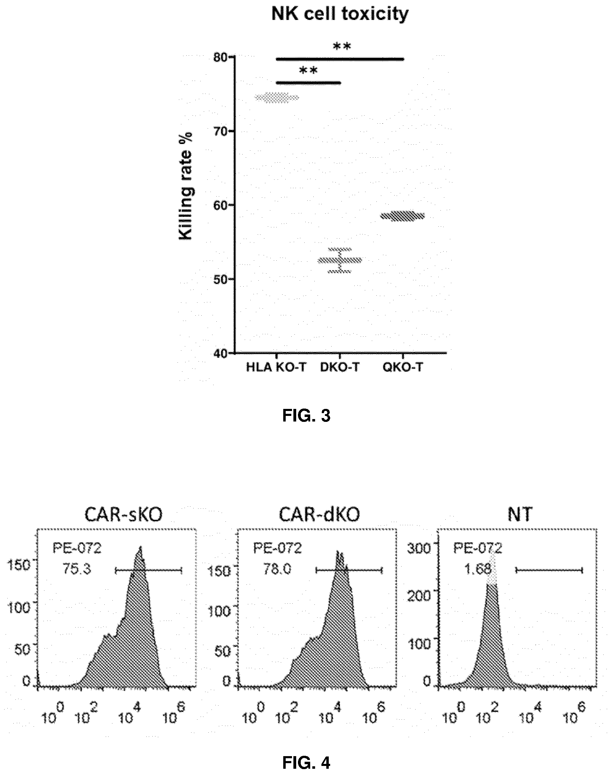

It can be seen that corresponding genes in the QKO-T cells and the KO-T cells prepared in the present disclosure are effectively knocked out. Then, the suppression effect of the KO-T cells and the QKO-T cells prepared in the above on the killing effect of the NK cells was detected according to the method described in Example 3, with results being shown in FIG. 3.

It can be seen that the QKO-T cells with the NK activating receptor binding molecule, the TCR gene and multiple MHC related genes being simultaneously knocked out likewise can significantly suppress the killing effect of the NK cells.

### Example 5. Construction of CAR-dKO T cells and verification of functions thereof

### 5.1 Construction of CAR-sKO T cells and CAR-dKO T cells

Sequences encoding the following proteins were synthesized, and cloned into the pLVX vector (Public Protein/Plasmid Library (PPL), Lot No.: PPL00157-4a): CD8α signal peptide (SEQ ID NO: 12), anti-CD19scFv(SEQ ID NO: 13), CD8α hinge region (SEQ ID NO: 14), CD8α transmembrane region (SEQ ID NO: 15), 4-1BB intracellular region (SEQ ID NO: 16), CD3ζ intracellular signaling domain (SEQ ID NO: 17), and correct insertion of target sequence was confirmed by sequencing.

After 3 ml of Opti-MEM (Gibco, Lot No. 31985-070) was added to a sterile tube to dilute the above plasmids, packaging vector psPAX2 (Addgene, Lot No. 12260) and envelope vector pMD2.G (Addgene, Lot No. 12259) were then added according to a ratio of plasmid: virus packaging vector: virus envelope vector = 4:2:1. Then, 120 µl of X-treme GENE HP DNA transfection reagent (Roche, Lot No. 06366236001) was added, the mixture was well mixed immediately, and incubated at room temperature for 15 min, and then the plasmid/vector/transfection reagent mixture was added dropwise into a culture flask of 293T cells. Viruses were collected at 24 and 48 hours and combined, and then subjected to ultracentrifugation (25000 g, 4 °C, 2.5 hours) to obtain concentrated lentiviruses.

Concentrated lentiviruses were transducted into sKO-T cells (only B2M being knocked out) and dKO-T cells (B2M/ICAM3 both being knocked out), to obtain CAR-sKO T cells and CAR-dKO T cells, respectively. Unmodified wild-type T cells served as negative control (NT).

After 11 days of culture at 37 °C and 5% CO₂, expression levels of scFv on the CAR T cells were detected by flow cytometry using Biotin-SP (long spacer) AffiniPure Goat Anti-Mouse IgG, F(ab')2 Fragment Specific (min X Hu, Bov, Hrs Sr Prot) (jackson immunoresearch, Lot No. 115-065-072) as a primary antibody and APC Streptavidin (BD Pharmingen, Lot No. 554067) or PE Streptavidin (BD Pharmingen, Lot No. 554061) as a secondary antibody, with results being shown in FIG. 4.

It can be seen that scFvs in the CAR T cells prepared in the present disclosure all can be effectively expressed.

### 5.2 Detecting killing effect of CAR-T cells on target cells

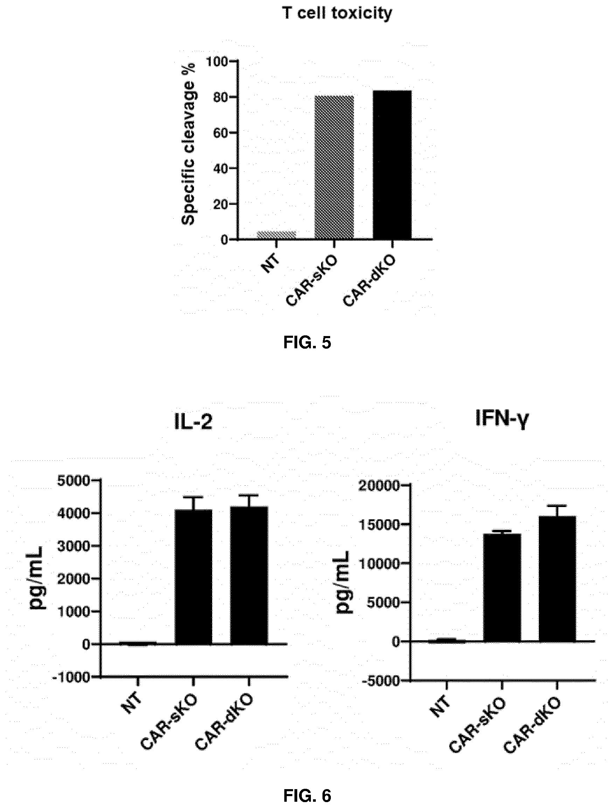

Nalm6 target cells carrying a fluorescein gene were first plated into a 96-well plate at a concentration of 1×10⁴ cells per well, then NT cells, CAR-sKO T cells and CAR-dKO T cells were plated into the 96-well plate at an effector-target ratio of 2:1 (i.e., a ratio of effector T cells to target cells) for co-culture, and the fluorescence value was measured with a plate reader 16-18 hours later. According to calculation formula: (mean value of fluorescence of target cell - mean value of fluorescence of sample)/mean value of fluorescence of target cell × 100%, the killing efficiency was calculated, with results being shown in FIG. 5.

It can be seen that the CAR-sKO T cells and the CAR-dKO T cells both have specific killing effects on the target cells, and further knocking out the NK activating receptor ICAM3 does not adversely affect the killing effect of the CAR-T cells.

### 5.3 Detecting the cytokine release level of the CAR-T cells

Target cells Nalm6 were plated in a 96-well plate at a concentration 1×10⁵ cells/well, then the NT cells, the CAR-sKO T cells and the CAR-dKO T cells of the present disclosure and the target cells were co-cultured at a ratio of 1:1. After 18-24 hours, cell co-culture supernatant was collected.

The 96-well plate was coated with capture antibody Purified anti-human IL2 Antibody (Biolegend, Lot No. 500302) or Purified anti-human IFN-γ Antibody (Biolegend, Lot No. 506502) and incubated overnight at 4 °C, then the antibody solution was removed, 250 µL of PBST (IXPBS containing 0.1% Tween) solution containing 2% BSA (sigma, Lot No. V9009333-1 kg) was added, followed by incubation at 37 °C for 2 hours. The plate was then washed 3 times with 250 µL of PBST (1XPBS containing 0.1% Tween). 50 µL of the cell co-culture supernatant or a standard was added to each well, followed by incubation at 37 °C for 1 hour, then the plate was washed 3 times with 250 µL of PBST (1XPBS containing 0.1% Tween). Then 50 µL of detection antibody Anti-Interferon gamma antibody [MD-1] (Biotin) (abcam, Lot No. ab25017) was added to each well, and after 1 hour of incubation at 37 °C, the plate was washed 3 times with 250 µL of PBST (1XPBS containing 0.1% Tween). HRP Streptavidin (Biolegend, Lot No. 405210) was then added, and after 30 minutes of incubation at 37 °C, the supernatant was discarded, 250 µL of PBST (1XPBS containing 0.1% Tween) was added, and the plate was washed 5 times. 50 µL of TMB substrate solution was added to each well. The reaction was carried out in the dark at room temperature for 30 minutes, after which 50 µL of 1 mol/L H₂SO₄ was added to each well to quench the reaction. Within 30 minutes after quenching the reaction, absorbance at 450 nm was detected by a plate reader, and the content of cytokines was calculated according to a standard curve (plotted according to the measured value and concentration of the standard), with results being shown in FIG. 6.

It can be seen that both the CAR-sKO T cells and the CAR-dKO T cells can significantly improve the cytokine release level of the CAR T cells on the target cells, and further knocking out the NK activating receptor ICAM3 does not adversely affect the cytokine release level of the CAR-T cells.

It should be noted that the above-mentioned are merely for preferred examples of the present disclosure and not used to limit the present disclosure. For a person skilled in the art, various modifications and changes could be made to the present disclosure. A person skilled in the art should understand that any amendments, equivalent replacements, improvements, and so on, within the spirit and principle of the present disclosure, should be covered within the scope of protection of the present disclosure.

## Claims

1. An engineered immune cell, wherein expression of at least one MHC related gene and at least one NK activating receptor binding molecule is suppressed or silenced, so as to suppress killing of the engineered immune cell by NK cells.

2. The engineered immune cell according to claim 1, wherein the MHC related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA and a combination thereof.

3. The engineered immune cell according to claim 2, wherein the MHC related gene is selected from the group consisting of HLA-A, HLA-B, HLA-C, B2M, RFX5, RFXAP, RFXANK, CIITA and a combination thereof.

4. The engineered immune cell according to claim 1, wherein the NK activating receptor binding molecule binds to an NK activating receptor selected from the group consisting of NKG2C, NKG2E, NKG2D, NKp30, NKp44, NKp46, NKp80, 2B4, DNAM-1, CD2 and LFA-1.

5. The engineered immune cell according to claim 1, wherein the NK activating receptor binding molecule is selected from the group consisting of MICA, MICB, ULBP1, ULBP2, ULBP3, ULBP4, ULBP5, ULBP6, Rae-1, H60, MULT1, B7-H6, BAG6, PfEMP1, HSPGS, AICL, CD112, CD155, CD48, CD58, CD59, ICAM1, ICAM2, ICAM3, STAT1, JAK1, IFNGR2, JAK2 and IFNGR1.

6. The engineered immune cell according to claim 5, wherein the NK activating receptor binding molecule is selected from the group consisting of CD112, CD155, CD48, MICA, MICB, CD58, B7-H6, ICAM1, ICAM2 and ICAM3.

7. The engineered immune cell according to any one of claims 1-6, wherein the engineered immune cell further comprises suppressed or silenced expression of at least one TCR/CD3 gene selected from the group consisting of TRAC, TRBC, CD3 γ, CD3 δ, CD3 ε, CD3 ζ and a combination thereof.

8. The engineered immune cell according to claim 7, wherein expression of at least one TCR/CD3 gene, at least one MHC related gene, and at least one NK activating receptor binding molecule of the engineered immune cell is suppressed or silenced, wherein the TCR/CD3 gene is selected from the group consisting of TRAC, TRBC, CD3 γ, CD3 δ, CD3 ε, CD3 ζ and a combination thereof, the MHC related gene is selected from the group consisting of HLA-A, HLA-B, HLA-C, B2M, RFX5, RFXAP, RFXANK, CIITA and a combination thereof, and the NK activating receptor binding molecule is selected from the group consisting of CD112, CD155, CD48, MICA, MICB, CD58, B7-H6, ICAM1, ICAM2, ICAM3 and a combination thereof.

9. The engineered immune cell according to any one of claims 1-8, wherein the engineered immune cell further comprises suppressed or silenced expression of one or more genes selected from the following: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3.

10. The engineered immune cell according to any one of claims 1-9, wherein the engineered cell further expresses an immune recognition receptor, which comprises a ligand binding domain, a transmembrane domain and an intracellular signaling domain.

11. The engineered immune cell according to claim 10, wherein the immune recognition receptor is a chimeric antigen receptor or a T cell receptor.

12. The engineered immune cell according to claim 11, wherein the immune recognition receptor is a chimeric antigen receptor, which comprises a ligand binding domain, a transmembrane domain, a costimulatory domain and an intracellular signaling domain.

13. The engineered immune cell according to claim 10, wherein the immune recognition receptor binds to a target selected from the group consisting of TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6/E7, MAGE A1, ETV6-AML, sperm protein 17, XAGE1, Tie 2, MAD-CT-1, MAD-CT-2, Fos-related antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D and any combination thereof.

14. The engineered immune cell according to any one of claims 1-13, wherein the immune cell is selected from the group consisting of a T cell, a macrophage, a dendritic cell, a monocyte, an NK cell or an NKT cell.

15. The engineered immune cell according to any one of claims 1-13, wherein the immune cell is a T cell selected from the group consisting of CD4+/CD8+ T cell, CD4+ helper T cell, CD8+ T cell, tumor infiltrating cell, memory T cell, naive T cell, γδ-T cell, or αβ-T cell.

16. The engineered immune cell according to any one of claims 1-15, wherein the immune cell is derived from adult stem cells, embryonic stem cells, umbilical cord blood stem cells, progenitor cells, bone marrow stem cells, induced pluripotent stem cells, totipotent stem cells or hematopoietic stem cells.

17. A pharmaceutical composition, comprising the engineered immune cell according to any one of claims 1-16 and one or more pharmaceutically acceptable excipients.

18. Use of the engineered immune cell according to any one of claims 1-16 in the preparation of a medicament for treatment of cancers, infection or autoimmune diseases.
